(19) Europäisches Patentamt
European Patent Office
Office européen des brevets

(11) Veröffentlichungsnummer: **0 044 972**
**A1**

(12) # EUROPÄISCHE PATENTANMELDUNG

(21) Anmeldenummer: **81105205.9**

(22) Anmeldetag: **04.07.81**

(51) Int. Cl.³: **C 07 C 69/736**, C 07 C 67/317

(30) Priorität: **29.07.80 DE 3028626**

(43) Veröffentlichungstag der Anmeldung: **03.02.82**
**Patentblatt 82/5**

(84) Benannte Vertragsstaaten: **BE CH DE FR GB LI**

(71) Anmelder: **BASF Aktiengesellschaft,**
**Carl-Bosch-Strasse 38, D-6700 Ludwigshafen (DE)**

(72) Erfinder: **Husslein, Gerd, Dr., Herrenbergstrasse 2,**
**D-6702 Bad Duerkheim (DE)**
Erfinder: **Hamprecht, Gerhard, Dr.,**
**Rote-Turm-Strasse 28, D-6940 Weinheim (DE)**

(54) **Verfahren zur Herstellung von 2-Aryloxy-acrylsäureverbindungen.**

(57) Herstellung von 2-Aryloxy-acrylsäureverbindungen durch Umsetzung von 2-Aryloxy-2-halogenpropionsäureverbindungen bei −10 bis +150 °C mit basischen Verbindungen.

Die nach dem Verfahren der Erfindung herstellbaren Endstoffe sind wertvolle Ausgangsstoffe für die Herstellung von Pharmazeutika, Pflanzenschutzmitteln und Farbstoffen.

EP 0 044 972 A1

ACTORUM AG

COMPLETE DOCUMENT

Verfahren zur Herstellung von 2-Aryloxy-acrylsäureverbindungen

Die Erfindung betrifft ein Verfahren zur Herstellung von 2-Aryloxy-acrylsäureverbindungen durch Umsetzung von 2-Aryloxy-2-halogenpropionsäureverbindungen bei -10 bis +150°C mit basischen Verbindungen.

Es ist aus Zhurnal Organicheskoi Khimii, Band 1, Seiten 475 bis 480 (englische Übersetzung Seite 467 bis 471) (1965) bekannt, daß man p-Chlorphenoxymalonsäure mit Formaldehyd und Aminen bei Raumtemperatur während 8 Stunden zu α-p-Chlorphenoxy-ß-alaninen umsetzt; als Nebenprodukt erhält man je nach dem verwendeten Amin α-(p-Chlorphenoxy)-acrylsäuren in Ausbeuten von 19 bis 55 Prozent.

Ebenfalls werden Phenoxyessigsäuren am Rückfluß in Gegenwart von Triäthylamin und Essigsäureanhydrid mit aromatischen Aldehyden zu 2-Aryloxy-acrylsäuren, z.B. in (Beispiel 1) 45 Prozent Ausbeute, kondensiert (FP-PS 1 583 931). Eine weitere Umsetzung mit Natriumphenolaten und α,α-Dichlorpropionsäureester, Abspaltung eines Phenolmoleküls aus den entstandenen Brenztraubensäurediphenylketalen im Hochvakuum durch Thermolyse (Ann. 709 (1967), 173 bis 184) ergibt enbenfalls vorgenannte Acrylsäureverbindungen.

Alle Verfahren sind im Hinblick auf leichte Zugänglichkeit, Lagerfähigkeit und leichte Handhabung der Ausgangsstoffe, einfachen und wirtschaftlichen Betrieb sowie Ausbeute und Reinheit des Endstoffs unbefriedigend.

Es wurde nun gefunden, daß man 2-Aryloxy-acrylsäureverbindungen der Formel

WB/BL

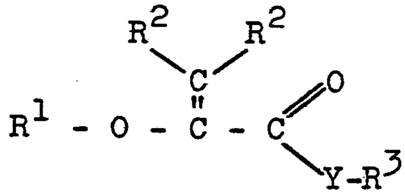
I,

worin Y ein Sauerstoffatom oder den Rest 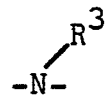 bedeutet, $R^1$ einen aromatischen Rest bezeichnet, die einzelnen Reste $R^2$ und $R^3$ gleich oder verschieden sein können und jeweils für ein Wasserstoffatom oder einen aliphatischen, cycloaliphatischen, araliphatischen oder aromatischen Rest stehen, vorteilhaft erhält, wenn man 2-Aryloxy-2-halogenpropionsäureverbindungen der Formel

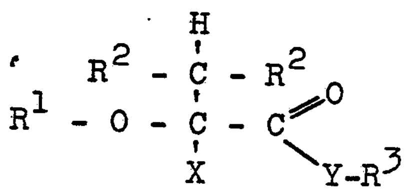
II,

worin $R^1$, $R^2$, $R^3$ und Y die vorgenannten Bedeutungen besitzen und X ein Chloratom oder ein Bromatom bezeichnet, mit einer basischen Verbindung bei einer Temperatur von -10 bis +150°C umsetzt.

Verwendet man 2-(4'-Chlorphenoxy)-2-brom-propionsäuremethylester und Triäthylamin als Ausgangsstoffe, kann der Reaktionsablauf des erfindungsgemäßen Verfahrens durch folgendes Formelschema wiedergegeben werden:

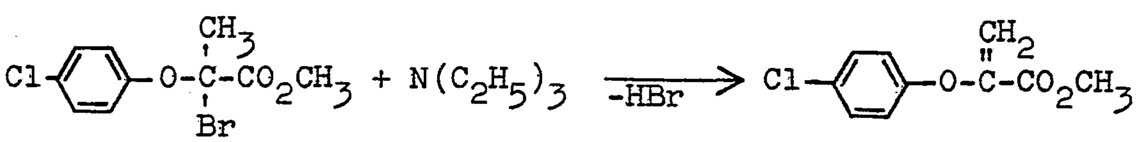

Im Vergleich zu den bekannten Verfahren liefert das Verfahren nach der Erfindung auf einfacherem und wirtschaftlicherem Wege 2-Aryloxy-acrylsäureverbindungen in besserer Ausbeute und Reinheit. Alle diese vorteilhaften Ergebnisse sind überraschend, denn es wird in der DE-OS 27 20.654 beschrieben, daß die Umsetzung von α-Halogenalkancarbonsäureverbindungen mit basischen Azolen, beispielsweise Imidazol, und basischen Stoffen, beispielsweise Triäthylamin, im Temperaturbereich von 0 bis 120°C zu Substitutionsprodukten und nicht zu ungesättigten Verbindungen führen (Beispiel 28).

Weiterhin ist aus Houben-Weyl, Methoden der Organischen Chemie, Band 6/3 (1965), Seite 237 bekannt, daß α-Halogenäther durch Alkoholate oder Phenolate substituiert werden ohne daß eine Eliminierung von Halogenwasserstoff erfolgt, auch wenn dies strukturell möglich wäre. Nach dieser Lehre erfolgt der Angriff einer Base an dem α-ständigen Kohlenstoffatom an dem das reaktive Halogen gebunden ist. Es ist daher überraschend, daß beim erfindungsgemäßen Verfahren die Base am zum Halogen ß-ständigen Kohlenstoffatom angreift und so die Eliminierung von Halogenwasserstoff bewirkt.

Bevorzugte Ausgangsstoffe II und dementsprechend bevorzugte Endstoffe I sind solche, in deren Formeln Y ein Sauerstoffatom oder den Rest $\overset{R^3}{\overset{|}{-N-}}$ bedeutet, $R^1$ einen Phenylrest oder einen durch 1, 2 oder 3 Chloratome, Bromatome, Alkylgruppen und/oder Alkoxygruppen mit jeweils 1 bis 6 Kohlenstoffatomen substituierten Phenylrest oder einen unsubstituierten oder in vorgenannter Weise substituierten Diphenylrest bezeichnet, die einzelnen Reste $R^2$ und $R^3$ gleich oder verschieden sein können und jeweils für ein Wasserstoffatom, einen Alkylrest mit 1 bis 18, insbesondere 1 bis 8 Kohlenstoffatomen, einen Cycloalkylrest mit 5 bis 8 Kohlenstoffatomen,

einen Aralkylrest oder Alkylarylrest mit 7 bis 12 Kohlenstoffatomen, einen Phenylrest stehen, X ein Chloratom oder ein Bromatom bedeutet. Die vorgenannten Reste können noch durch unter den Reaktionsbedingungen inerte Atome oder Gruppen, z.B. Alkylgruppen oder Alkoxygruppen mit jeweils 1 bis 4 Kohlenstoffatomen, Nitrogruppen, Cyangruppen, Arylreste substituierende Jodatome, Fluoratome, Chloratome, Bromatome und/oder Trifluormethylgruppen, substituiert sein.

So kommen folgende Ausgangsstoffe II in Betracht: 2-Phenoxy-2-chlorpropionsäure und in 3-Stellung einfach oder gleich oder verschieden zweifach durch die Methyl-, Äthyl-, n-Propyl-, iso-Butyl-, sek.-Butyl-, tert.-Butyl-, n-Pentyl-, 2'-Pentyl-, 3'-Pentyl-, 4'-Methyl-3'-pentyl-, 1'-Chloräthyl-, Trifluormethyl-, Trichlormethyl-, Difluormethyl-, Nitromethyl-, Cyanomethyl-, 2-Chloräthyl-, 1-Chlorpropyl-, 2'-Chlorpropyl-, 1'-Chlor-2'-propyl-, 1'-Fluoräthyl-, 2'-Fluoräthyl-, 1'-Fluor-2'-propyl-, 3'-Brombutyl-, 1',1',1'-Trifluorisopropyl-, Methoxyäthyl-, Methoxyisopropyl-, Äthoxy-tert.-butyl-, Methoxy-tert.-butyl-, Cyclopentyl-, Cyclohexyl-, 2'-Chlorcyclohexyl-, 2'-(Trifluormethyl)-cyclohexyl-, Benzyl-, Phenyl-, α-Phenyläthyl-gruppe, in o-, m-, p-Stellung einfach oder in 2', 4'-, 2',3'-, 2',6'-, 2',5'-, 3',4'-, 4',5'-Stellung gleich oder unterschiedlich 2fach durch Chloratome, Bromatome, Fluoratome, Methyl-, Äthyl-, Propyl-, iso-Propyl-, Butyl-, iso-Butyl-, sek.-Butyl-, tert.-Butyl-, Methoxy-, Äthoxy-, Propoxy-, iso-Propoxy-, Butoxy-, iso-Butoxy-, sek.-Butoxy-, tert.-Butoxy-, Phenyl-, Trifluormethyl-gruppen substituierte Phenylgruppen substituierte 2-Phenoxy-2-chlorpropionsäuren; vorgenannten Säuren homologe unsubstituierte oder in vorgenannter Weise substituierte Methyl-, Äthyl-, Propyl-, iso-Propyl-, Butyl-, iso-Butyl-, sek.-Butyl-, tert.-Butyl-, Cyclohexyl-, Cyclopentyl-, Phenyl-, Benzyl-ester; homologe unsubstituierte oder in vorgenannter

Weise substituierte einfach oder zweifach gleich oder unterschiedlich durch die Methyl-, Äthyl-, Propyl-, iso-Propyl-, Butyl-, iso-Butyl-, sek.-Butyl-, tert.-Butyl-, Cyclohexyl-, Phenyl-, Benzyl-gruppe am Stickstoffatom substituierte Propionsäureamide; homologe unsubstituierte oder in vorgenannter Weise substituierte in 2'-, 3'-, 4'-, 5'-, 6'-Stellung am Phenylkern einfach oder gleich oder verschieden 2fach oder 3fach durch Fluoratome, Chloratome, Bromatome, Jodatome, Trifluormethyl-, Methyl-, Äthyl-, Propyl-, iso-Propyl-, Butyl-, iso-Butyl-, sek.-Butyl-, tert.-Butyl-, Methoxy-, Äthoxy-, Propoxy-, iso-Propoxy-, Butoxy-, iso-Butoxy-, sek.-Butoxy-, tert.-Butoxy-, Nitro-, Phenyl-gruppen substituierte 2-Phenoxyverbindungen; homologe 2-Bromverbindungen.

Die Ausgangsstoffe II können durch die in der deutschen Offenlegungsschrift 27 20 654 beschriebene Arbeitsweise durch Umsetzung von Aryloxyalkancarbonsäurederivaten der Formel

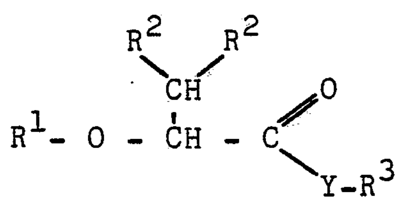
III,

worin Y, $R^1$, $R^2$ und $R^3$ die vorgenannten Bedeutungen besitzen, mit N-Bromsuccinimid oder N-Chlorsuccinimid in Gegenwart eines Lösungsmittels wie Tetrachlorkohlenstoff und eines Radikalbildners wie beispielsweise Dibenzoylperoxid erhalten werden.

Besonders bevorzugte Ausgangsstoffe II sind: 2-Brom-2-(2',4'-dichlorphenoxy)-propionsäuremethylester, 2-Brom-2-(4'-chlorphenoxy)-propionsäureäthylester, 2-Brom-2-(3'-trifluormethylphenoxy)-propionsäuremethylester, 2-Chlor-2-(2',4'-dichlorphenoxy)-propionsäuremethylester, 2-Brom-2-

-(4'-chlorphenoxy)-buttersäureäthylester, 2-Brom-2-(4'-fluorphenoxy)-propionsäuremethylester, 2-Brom-2-(2'-brom-4'-chlorphenoxy)-propionsäuremethylester, 2-Brom-2-(2',4'-dichlorphenoxy)-propionsäureisooctylester, 2-Brom-2-(2',4',5'-trichlorphenoxy)-propionsäure-tert.-butylester, 2-Brom-2-(4'-phenylphenoxy)-propionsäuremethylester, 2-Brom-2-(4'-nitrophenoxy)-propionsäureäthylester, 2-Brom-2-(2',4'-dichlorphenoxy)-iso-valeriansäureäthylester, 2-Brom-2-(3'-trifluormethylphenoxy)-propionsäure-p-chlorphenylester, 2-Brom-2-(2',4'-dichlorphenoxy)-propionsäure-N,N-dimethylamid.

Die Umsetzung wird bei einer Temperatur von -10 bis +150°C, vorzugsweise von 0 bis +120°C, insbesondere von +10 bis +100°C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogen kohlenwasserstoffe, insbesondere Chlorkohlenwasserstoffe, z.B. Tetrachloräthylen, 1,1,2,2- oder 1,1,1,2-Tetrachloräthan, Amylchlorid, Dichlorpropan, Methylenchlorid, Dichlorbutan, Isopropylbromid, Chloroform, Äthyljodid, Propyljodid, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol; Ketone wie Methyläthylketon, Aceton, Diisopropylketon, Diäthylketon, Methylisobutylketon, Mesityloxid, Acetophenon, Cyclohexanon, Äthylisoamylketon, Diisobutylketon, Methylcyclohexanon, Dimethylcyclohexanon; Ester wie Methylacetat, n-Propylacetat, Methylpropionat, Butylacetat, Äthylformiat, Phthalsäuremethylester, Benzoesäuremethylester, Essigester, Phenylacetat; Nitrile wie Acetonitril, Butyronitril, Isobutyronitril,

Benzonitril, m-Chlorbenzonitril; Sulfoxide wie Dimethylsulfoxid, Diäthylsulfoxid, Dimethylsulfon, Diäthylsulfon, Methyläthylsulfon, Tetramethylensulfon; aliphatische oder cycloaliphatische Kohlenwasserstoffe, z.B. Heptan, α-Pinen, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan,2,2,4-Trimethylpentan, 2,2,3-Trimethylpentan, 2,3,3-Trimethylpentan, Octan; N,N-disubstituierte Säureamide wie Dimethylformamid, Dimethylacetamid; und entsprechende Gemische. Zweckmäßig verwendet man das Lösungsmittel in einer Menge von 100 bis 10 000 Gewichtsprozent, vorzugsweise von 500 bis 2 000 Gewichtsprozent, bezogen auf Ausgangsstoff II.

Die Umsetzung wird in Gegenwart einer basischen Verbindung, vorteilhaft in einer Menge von 0,8 bis 2, vorzugsweise von 1 bis 1,5 Äquivalenten basischer Verbindung, bezogen auf ein Mol Ausgangsstoff II, durchgeführt. Es können anorganische oder organische Basen, z.B. Zinkverbindungen, primäre, sekundäre Amine, Erdalkaliverbindungen, Ammoniumverbindungen, Alkaliverbindungen sowie entsprechende Gemische verwendet werden. Bevorzugte basische Verbindungen sind Aluminium-, Erdalkali- und Alkalialkoholate und tertiäre Amine. Es kommen z.B. als basische Verbindungen in Frage: Aluminiumisopropylat, Kalium-tert.-butylat, Magnesium-cyclohexanolat, Natrium-isobutylat, Natrium-isoamylat, Natriummethylat, Natriumäthylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumäthylat, Kalium-n-propylat, Kaliumisopropylat, Kalium-n-butylat, Kalium-isobutylat, Kalium-sek.-butylat, Kalium-tert.-butylat, Kaliumäthylenglykolat, Kaliumpropylen-(1,2)-glykolat, Kaliumpropylen-

-(1,3)-glykolat, Kaliumdiäthylenglykolat, Kaliumtriäthylenglykolat, Kaliumdipropylen-(1,2)-glykolat; Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Dipropyl-p-aminopyridin, N,N-Dimethylaminoäthanol, N,N-Diäthylaminoäthanol, N,N-Dipropylaminoäthanol, N-Methylpyrrolidon, N-Äthylpyrrolidon, N-Methylpiperidin, N-Äthylpiperidin, N-Methylpyrrolidin, N-Äthylpyrrolidin, N-Methylimidazol, Pyridin, Chinolin, α-Picolin, ß-Picolin, γ-Picolin. Man kann gegebenenfalls die basische Verbindung als Lösungsmittel bzw. Verdünnungsmittel, vorteilhaft in einem solchen Falle zusätzlich in einer Menge von 0,5 bis 10, vorzugsweise von 1 bis 5 Äquivalenten basischer Verbindung, bezogen auf ein Mol Ausgangsstoff II, verwenden.

Die Reaktion kann wie folgt durchgeführt werden: Ein Gemisch von Ausgangsstoff II und basischer Verbindung, gegebenenfalls zusammen mit Lösungsmittel, wird während 0,5 bis 20 Stunden bei der Reaktionstemperatur gehalten. Aus dem Reaktionsgemisch wird der Endstoff I in üblicher Weise, z.B. durch Filtration und fraktionierte Destillation, abgetrennt.

In einer bevorzugten Form des erfindungsgemäßen Verfahrens legt man den Ausgangsstoff II gegebenenfalls in einem Lösungsmittel vor und gibt gegebenenfalls unter Kühlung die Base allein oder mit dem gewählten Lösungsmittel verdünnt zu und führt die Reaktion bei +10 bis +100°C, während 0,5 bis 20 Stunden, drucklos durch.

Die nach dem Verfahren der Erfindung herstellbaren Endstoffe I sind wertvolle Ausgangsstoffe für die Herstellung

von Pharmazeutika, Pflanzenschutzmitteln und Farbstoffen. Bezüglich der Verwendung wird auf die vorgenannten Veröffentlichungen verwiesen.

Die in den folgenden Beispielen aufgeführten Teile bedeuten Gewichtsteile.

Beispiel 1

Zu einer Lösung von 65,6 Teilen 2-Brom-2-(2',4'--dichlorphenoxy)-propionsäuremethylester in 500 Teilen Dichlormethan gibt man bei 15°C 21 Teile Triäthylamin und rührt bei 22°C 12 Stunden nach. Das ausgefallene Salz wird abgesaugt, das Filtrat zweimal mit Wasser gewaschen und getrocknet. Nach dem Abziehen des Lösungsmittels wird der Rückstand destilliert. Man erhält 40,6 Teile (82 % der Theorie) 2-(2',4'-Dichlorphenoxy)-acrylsäuremethylester vom Kp 117°C (0,1 mbar).

Beispiel 2

Die Umsetzung wird analog Beispiel 1 mit 74,5 Teilen 2-Brom-2-(3'-trifluormethylphenoxy)-propionsäuremethylester in 200 Teilen Tetrachlorkohlenstoff und 30 Teilen Triäthylamin durchgeführt. Man rührt 8 Stunden bei 22°C. Nach dem Absaugen wird das Filtrat eingeengt und der Rückstand destilliert. Man erhält 55 Teile (74,5 % der Theorie) 2-(3'-Trifluormethylphenoxy)-acrylsäuremethylester vom Kp 58°C/0,1 mbar.

Beispiel 3

Die Umsetzung wird analog Beispiel 1 mit 39,6 Teilen 2-Brom-2-(4'-fluorphenoxy)-propionsäuremethylester in 200 Teilen Tetrachloräthylen und 25,8 Teilen N-Äthyl-N,N-diisopropylamin durchgeführt. Man rührt bei 22°C 8 Stunden.

Anschließend saugt man ab, wäscht das Filtrat, trocknet und engt ein. Der Rückstand liefert bei der Destillation 60 Teile (75 % der Theorie) 2-(4'-Fluorphenoxy)-acrylsäuremethylester vom Kp 79 bis 81°C/0,5 mbar.

Beispiel 4

Zu einer Lösung von 22,6 Teilen Kalium-tert.-butanolat in 200 Teilen tert.-Butanol gibt man 68,2 Teile 2-Brom-2-(2',4'-dichlorphenoxy)-propionsäure-N,N-dimethylamid und rührt bei 22°C 10 Stunden. Danach gießt man die Reaktionsmischung in Wasser ein und extrahiert den Endstoff mit Essigester. Nach dem Trocknen und Einengen erhält man 34,4 Teile (66 % der Theorie) 2-(2',4'-Dichlorphenoxy)-acrylsäure-N,N-dimethylamid.

BAD ORIGINAL

## Patentanspruch

Verfahren zur Herstellung von 2-Aryloxy-acrylsäureverbindungen der Formel

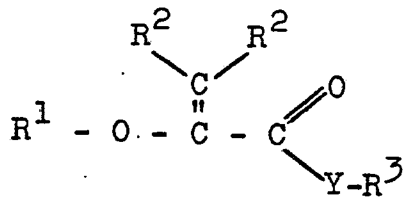

I,

worin Y ein Sauerstoffatom oder den Rest 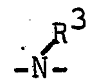 bedeutet, $R^1$ einen aromatischen Rest bezeichnet, die einzelnen Reste $R^2$ und $R^3$ gleich oder verschieden sein können und jeweils für ein Wasserstoffatom oder einen aliphatischen, cycloaliphatischen, araliphatischen oder aromatischen Rest stehen, dadurch gekennzeichnet, daß man 2-Aryloxy-2-halogenpropionsäureverbindungen der Formel

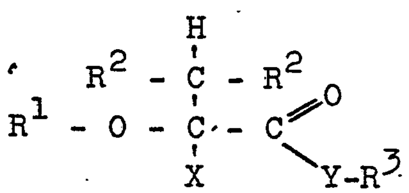

II,

worin $R^1$, $R^2$, $R^3$ und Y die vorgenannten Bedeutungen besitzen und X ein Chloratom oder ein Bromatom bezeichnet, mit einer basischen Verbindung bei einer Temperatur von -10 bis +150°C umsetzt.

Europäisches Patentamt

# EUROPÄISCHER RECHERCHENBERICHT

0044972

Nummer der Anmeldung
EP 81 10 5205

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | HOUBEN-WEYL: "Methoden der Organischen Chemie", Band V/1b, 1972, Georg Thieme Verlag Stuttgart, DE. * Seite 139, Abschnitt 2; Seite 155, Abschnitt 2 * | 1 |

----

**KLASSIFIKATION DER ANMELDUNG (Int. Cl.³)**

C 07 C 69/736
67/317

**RECHERCHIERTE SACHGEBIETE (Int. Cl.³)**

C 07 C 69/736
67/317

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 28-10-1981 | KINZINGER |

EPA form 1503.1 06.78